Europäisches Patentamt

European Patent Office

Office européen des brevets

(19)

(11) Publication number: 0 322 084
A2

# (12) EUROPEAN PATENT APPLICATION

(21) Application number: 88305198.9

(22) Date of filing: 08.06.88

(51) Int. Cl.⁴: **C12P 21/02 , C12N 15/00 , C07H 21/04 , C12N 5/00 , A61K 37/02**

(30) Priority: 22.12.87 JP 322843/87

(43) Date of publication of application:
28.06.89 Bulletin 89/26

(84) Designated Contracting States:
**BE CH DE ES FR GB IT LI NL SE**

(71) Applicant: **THE GREEN CROSS CORPORATION**
**15-1, Imabashi-1-chome Higashi-ku**
**Osaka-shi**
**Osaka 541(JP)**

(72) Inventor: **Noda, Munehiro**
**515, Tacho**
**Tenri-shi(JP)**
Inventor: **Ishikawa, Hideyuki**
**33-402, Senrioka Naka**
**Suita-shi(JP)**
Inventor: **Sukenobu, Yuriko Shin-Osaka**
**Tezuka Haitsu 612**
**5-15, Miyahara-5-chome Yodogawa-ku**
**Osaka(JP)**
Inventor: **Masaki, Atusi**

**Kori Danchi Cl-4 3-1, Korigaoka-5-chome**
**Hirakata-shi(JP)**
Inventor: **Arimura, Hirofumi**
**18-1-401 Uenosaka-2-chome**
**Toyonaka-shi(JP)**
Inventor: **Takechi, Kazuo**
**25-308,Kawanakashinmachi**
**Daito-shi(JP)**
Inventor: **Tsujikawa, Muneo**
**Kuzuha Manshon 302 26, Kitafunahashicho**
**Hirakata-shi(JP)**
Inventor: **Ishida, Yutaka**
**Shirakawa Manshon 206 5-12,**
**Makinokitamachi**
**Hirakata-shi(JP)**
Inventor: **Kawabe, Haruhide**
**29-14, Yamadanishi-3-chome**
**Suita-shi(JP)**

(74) Representative: **Ford, Michael Frederick et al**
**MEWBURN ELLIS & CO. 2/3 Cursitor Street**
**London EC4A 1BQ(GB)**

(54) **Tumor cell inhibition factor.**

(57) A tumor cell inhibition factor, which is a glycoprotein having single band of molecular weight of about 36,000 daltons is produced from culture liquid of human-origin fibroblasts and purified by collecting substances of molecular weight of about 10,000 and more followed by an ion-exchange method, chromatography and gel filtration. The DNA sequence coding the factor is determined, and an expression vector capable of expressing the DNA sequence and recombinant host cells transformed with the vector are prepared.

EP 0 322 084 A2

# TUMOR CELL INHIBITION FACTOR

This invention relates to a novel factor which exerts an inhibitory effect on tumor cells.

The existence of a tumor cell inhibition factor in the human fibroblast culture fluid is disclosed in JP-A-88423/84 and JP-A-18721/86. The product in the former reference is called Tumor-degenerating factor (TDF) and is a glycoprotein of molecular weight of 145,000, and that in the latter is called fibroblast-originating necrotic factor (FNF) having a molecular weight of about 10,000 obtained from human fibroblasts transformed into tumor cells (SV 40 transformed cell).

The object of this invention is to provide a novel tumor cell inhibition factor which can be obtained from human-origin fibroblasts, and which may occasionally be referred to FTX hereinafter.

The novel tumor cell inhibition factor of the present invention is prepared by the following method.

Preparation of the present FTX from an established cell line from a human kidney.

The process comprises the following steps:

cultivating an established cell line obtained from a human kidney,

removing contaminants from the supernatant of the culture fluid and obtaining a fraction containing a substance having a molecular weight of 10,000 or more,

dialysing the fraction obtained and passing through of anion exchanger,

subjecting the unabsorbed fraction to a chromatofocussing treatment on an anion exchanger, collecting a fraction having a molecular weight of 10,000 or more from the unabsorbed fraction, for example, by subjecting the unabsorbed fraction to ultrafiltration on a membrane of molecular weight cut-off level of 10,000, and subjecting it to gel filtration by high performance liquid chromatography (for example, on a TSK GEL 3000 and 2000 SW column at a pH of 7.0, attained for example by equilibration with a phosphate buffer solution containing sodium chloride) to obtain an active protein peak between molecular weight of 67 K and 32 K.

The active protein thus obtained may be purified further by subjecting its fraction to a reverse phase high performance liquid chromatography using for example a PR-304 column.

The above method of isolation of the present invention is explained in more detail in sections (1)-(3) below, further sections describing the characteristics of a protein isolated by the above method and how they were confirmed (4), sequencing of DNA (5), formation of an expression vector (6) and transformation of host cells (7).

## (1) Preparation of starting cells

Cells of a human-origin fibroblast strain established by cultivation are used as starting cells. An example of preferable starting cells is an established cell line of human kidney origin. A typical example is one which has been established by subculturing a primary culture or diploid cell obtained from a fetal human kidney.

## (2) Culture conditions

The media for cell cultivation which may be used in this invention are serum-free media such as Waymouth's medium and Dulbecco's modified MEM medium, preferably a serum-free medium supplemented with a proper amount (0,05 to 0.2% (w/v)) of human serum albumin or a medium supplemented with a low concentration (0.05 to 0.2% (w/v)) of serum. The medium may further be supplemented with lactalbumin hydrolysate, transferin, various kind of amino acids, various aliphatic acids, and hormones such as insulin.

The symbol "% (w/v)" means herein a percentage of a solute by weight per solution volume.

Preferably, air (a mixture of 95% of air and 5% of $CO_2$) is introduced into the medium in a suitable amount (flow rate: 10 to 500 ml/minute). A cultivation temperature of 20 to 37°C is preferable. The culture fluid is exchanged approximately every 2 to 3 days.

## (3) Purification

Since the substance according to this invention is present in the culture supernatant, it is purified, after removal of cells, based on its physico-chemical and bio-chemical properties. For example, the purification may be conducted by proper combination of concentration, treatment by ion exchangers, a fractionation based on the molecular weight, etc.

More specifically, it can be recovered in the following manner.

First, the cultured medium is centrifuged (for example, at 1500 rpm for 5 minutes) to recover supernatant.

The supernatant is treated with a cation exchanger to obtain an unadsorbed fraction. An example of suitable cation exchangers for the treatment is the CM-exchanger (CM-Sephadex). The carrier is equilibrated with a buffer solution of a pH of 5-6 and an ionic strength of 0.1-0.2M and is then brought in contact with a solution containing the above-mentioned culture supernatant, whereby contaminants are adsorbed onto the exchanger, to recover an unadsorbed fraction.

The unadsorbed fraction is then treated with an anion exchanger to obtain an unadsorbed fraction.

An example of anion exchangers suitable for the treatment is the DEAE exchanger (DEAE-Sepharose), and the fraction unadsorbed by the treatment is collected as follows. The fraction unadsorbed by CM-Sephadex is treated with an ultrafiltration membrane of molecular weight cut-off level of 10,000 (e.g., PelliconTM, mfd, by Millipore Co.) to obtain a high molecular weight fraction. The fraction thus obtained is concentrated and then dialyzed against, for example, a 50 mM tris-HCl buffer solution (pH 7.0). The resulting dialysate is introduced into a DEAE-Sepharaose column equilibrated with the same buffer solution and then the same buffer solution is passed through the column to collect the eluent. The unadsorbed fraction thus collected is then concentrated. Similar procedure is repeated three times for the concentration to obtain a concentrated solution of the unadsorbed fraction. The concentrated solution is then subjected to chromato-focussing on an anion exchanger, for example Poly buffer exchanger, to obtain an unadsorbed fraction.

The chromatofocussing treatment is carried out as follows. The concentrated solution obtained by the DEAE exchanger treatment is dialyzed against a 25 M triethylamine-HCl buffer solution (pH 11), the dialysate is introduced into a PolybufferTM Exchanger PBE 118 column equilibrated with the same buffer solution, then the same buffer solution is passed through the column, and the eluent is collected to obtain an unadsorbed fraction.

The unadsorbed fraction is then subjected to gel filtration in the following manner to obtain an active protein fraction.

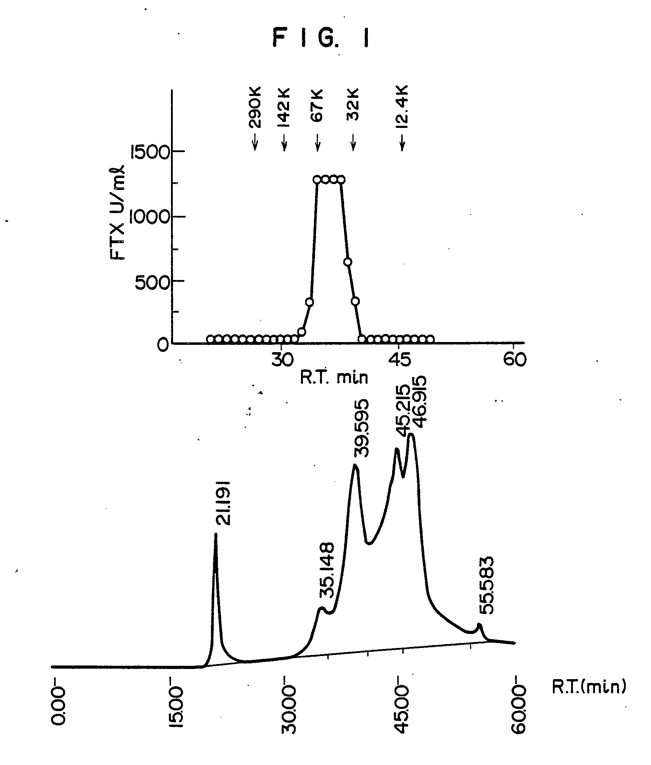

The fraction unadsorbed by the Polybuffer Exchanger PBE-118 column is treated with an ultrafiltration membrane of molecular weight cut-off level of 10,000 (e.g., PM-10TM, mfd, by Amicon Co.) to collect a high molecular weight fraction. The fraction is concentrated and subjected to gel filtration by HPLC (high performance liquid chromatography) using a TSK GEL 3000 and 2000 SW column equilibrated with a 0.1 M phosphate buffer solution (pH 7.0) containing 0.15 M NaCl, whereby a protein peak with attendant activity is obtained between molecular weight markers, Enolase (MW 67 K) and Adenylate Kinase (MW 32 K) (see Fig. 1).

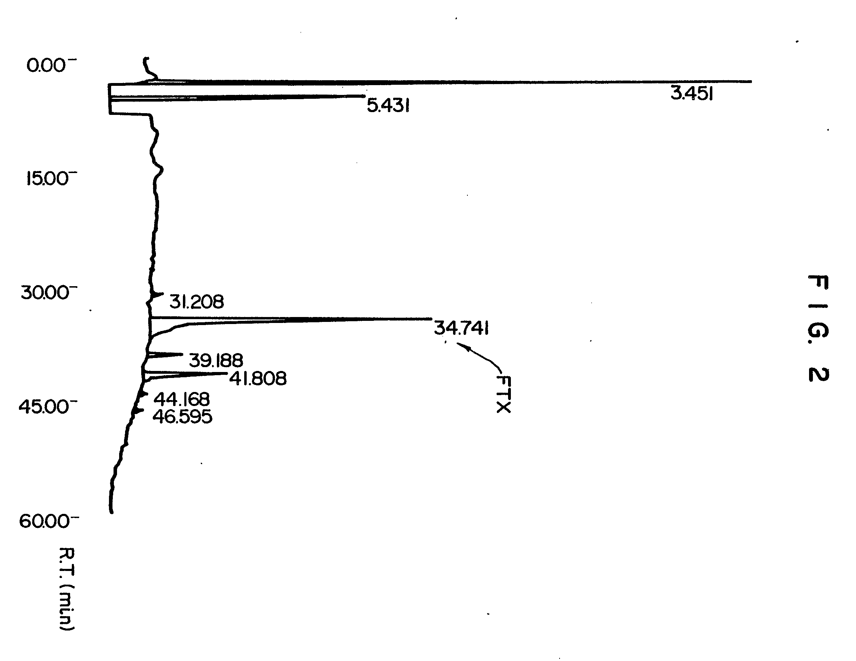

Then, the active fraction is subjected to a reverse phase HPLC (high performance liquid chromatography) in the following manner to obtain a purer product. Thus, the active fractions were collected and subjected to a reverse phase HPLC using a PR-304 column (chromatography conditions: A-solution ... 0.1% trifluoroacetic acid; B-solution... A-solution containing 80% acetonitrile; A-solution → B-solution 60 minutes linear gradient) to obtain the purer product (see Fig. 2). The product has a specific activity (U/mg) of 290,000. It was obtained in an amount of about 0.126 mg in terms of protein from 447,000 mg of protein of the fraction unadsorbed by CM-Sephadex.

The tumor cell inhibition substance thus obtained is a novel substance as judged from its characteristic properties described below. It may also be used as a reagent for biochemistry and pharmacology. For use as a medicinal agent it may be subjected, as described, to sterilization, asepsis, and medical preparation-forming processes according to conventional methods for medicine preparation. Thus, medicinal agents containing a novel tumor cell inhibition substance are provided.

(4) Characteristic properties of the substance of this invention

(a)(i) Biological activity:

It exerts growth inhibition and degeneration effects at least on KB cells, of rhinopharyngeal cancer origin, of strain ATCC CCL-17.

Details of procedure:

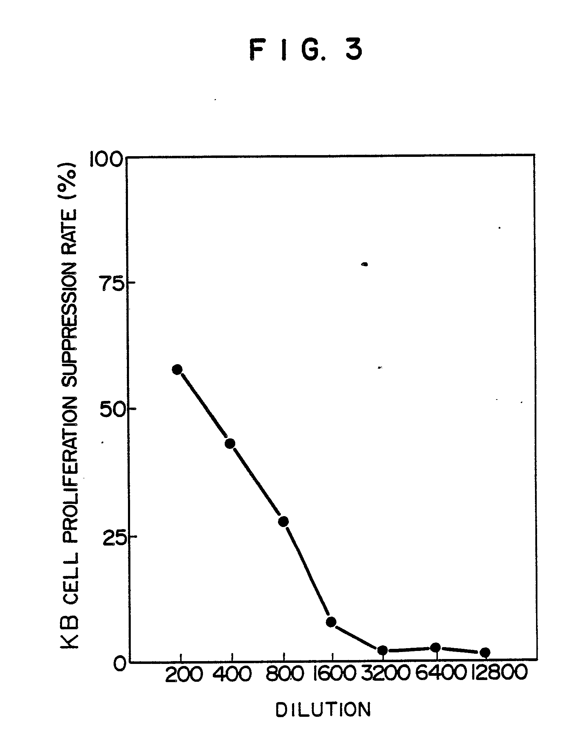

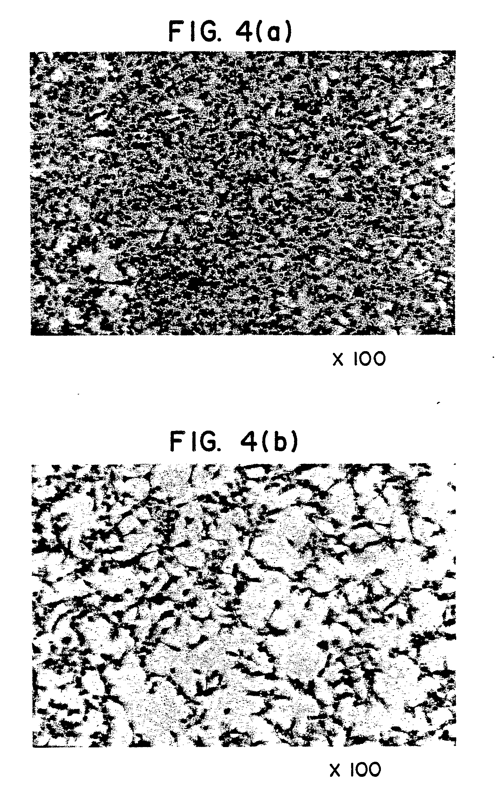

The sample of the substance isolated by the above method of this invention (hereinafter referred to as FTX) was subjected to two-times step dilution by the use of a 96-well microplate (50 $\mu$l/well). A KB cell fluid containing 10 x 10$^4$ cells/ml (human rhinopharyngeal cancer origin, 50 $\mu$l/well) was added thereto and cultivated at 37°C in an atmosphere containing 5% of $CO_2$ for 7 days. After cultivation, the supernatant was discarded and the remaining cells were stained with a 0.05% crystal violet staining solution. The amount of dye incorporated into living cells was determined from the absorbance at 590 nm by using Multiscan (mfd. by Titertec. Co.). The proportion of the absorbance of the treated cell of the FTX sample to the absorbance of the untreated cell was determined and was defined as growth rate. The dilution at which the FTX sample showed 50% growth inhibition was used to represent the FTX activity (U/ml). Fig. 3 shows the growth inhibition effect of FTX on KB cells (rhinopharyngeal cancer cells, ATCC-CCL-17). Fig. 4 shows the cancer cell degeneration effect obtained by FTX addition.

(ii) Further biological activity:

The FTX isolated by the above method exerted no effect on the NBT-reducing ability of ML-1 cells. Thus, it shows no differentiation-inducing ability.

It did not suppress the proliferation of virus in the FL-Sindbis virus system and exerted no inhibitory effect of L cells.

(b) Molecular weight:

single band of 36,000±1,000 daltons as determined by electrophoresis using SDS/polyacrylamide gel under reduced and nonreduced conditions.

Details of procedure:

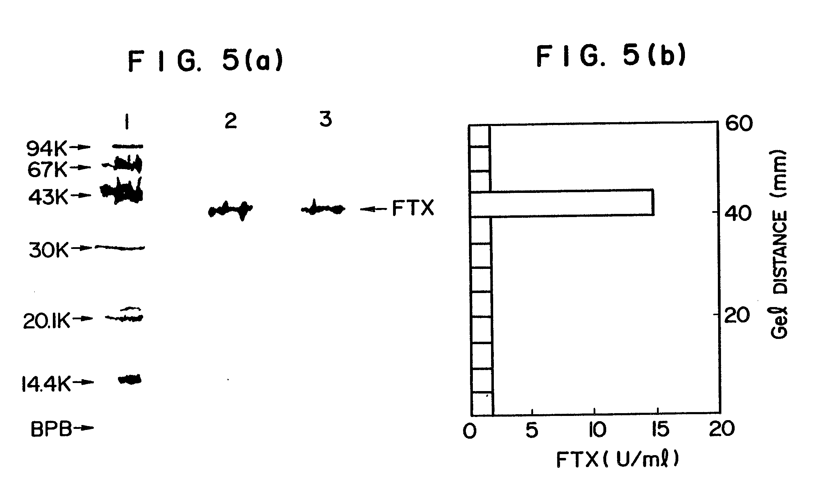

According to the method of Laemmli [Nature, 227, 680 (1970)], 0.3 $\mu$g of the FTX dissolved in Tris-buffer solution containing 2% SDS to which 2% of 2-mercaptoethyl alcohol had been added or not added were each subjected to electrophoresis using acrylamide gel containing 0.1% SDS. At the same time, for activity determination, 10 $\mu$l of concentrated solution (corresponding to 96 U) of a FTX sample which had been purified by the step of gel filtration by HPLC using TSK gel G3000 - 2000 SW column was subjected to electrophoresis. After electrophoresis, the gel was sliced to a thickness of 5 mm. The gel slices were each extracted at 4°C overnight in 1 ml of a medium for activity determination and the biological activity in respective slices was determined. The electrophoresis was conducted at 30 mA for about 4 hours. A standard molecular weight curve was prepared by using a standard molecular weight kit (mfd. by Pharmacia Co.). The molecular weight of the FTX of this invention was determined from its biological activity and its mobility in electrophoresis ascertained by means of protein argentation image. The results thus obtained are shown in Fig. 5. The symbols in Fig. 5 mean the following.

94 K (phosphorylase b), 67 K (bovine serum albumin), 43 K (ovalbumin), 30 K (carbonic anhydrase), 20.1 K (soybean trypsin inhibitor), 14.4 K ($\alpha$-lactalbumin).

The FTX isolated by the abovementioned method of this invention had a molecular weight of about 36,000 ± 1,000 as judged from the mobility of the molecular weight markers.

(c) Isoelectric point: pI of at least 10.5.

The isoelectric point of the FTX isolated by the abovementioned process of this invention was determined by preparative flat-bed electrofucussing (KLB) according to the method described in Biochem. Biophys. Acta., 194, 335 (1969) and Acta. Chem. Scand., 20, 820 (1966). It was found that the FTX of this invention had a pI of 10.5 or more.

(d) i) Amino acid composition and ii) amino sugar.

(i) Amino acid composition:

| AMINO ACID | P mole | mole % |
|---|---|---|
| Asp | 773.56 | 9.25 |
| Glu | 395.42 | 4.72 |
| Ser | 947.61 | 11.32 |
| Gly | 447.52 | 5.35 |
| His | 284.40 | 3.39 |
| Arg | 354.72 | 4.24 |
| Thr | 806.66 | 9.64 |
| Ala | 981.16 | 11.72 |
| Pro | 308.71 | 3.69 |
| Tyr | 305.52 | 3.66 |
| Val | 603.42 | 7.20 |
| Met | 337.81 | 4.04 |
| Cys | ND | - |
| Ile | 360.89 | 4.31 |
| Leu | 558.08 | 6.67 |
| Phe | 494.43 | 5.90 |
| Trp | ND | - |
| Lis | 412.36 | 4.92 |
| TOTAL | 8372.27 | 100.00 |

Applied FTX: 5 μg
ND: not determined

Detailed confirmation procedure:

A 5 μg portion of the FTX of this invention was hydrolyzed by treating it with 6N-HCl for 20 hours. The amino acid composition was then determined by the PICO-TAG™ method.

(ii) Amino sugar:

The protein isolated by the abovementioned method is a glycoprotein.

Detailed confirmation procedure:

A 5 μg portion of the FTX isolated by the above method was hydrolyzed by treating it with 4 N-HCl for 4 hours and then the analysis of amino sugar was carried out in a similar manner to that mentioned above, to detect glucosamine. From this result it was confirmed that the FTX isolated by the above method of this invention is a glycoprotein.

(e) N-terminal amino acid sequence

| 1 | 2 | 3 | 4 | 5 | 6 |
|---|---|---|---|---|---|
| Ala | Met | Phe | Met | Val | Lys |
| 7 | 8 | 9 | 10 | 11 | 12 |
| Asn | Gly | Asn | Gly | Thr | Ala |
| 13 | 14 | 15 | 16 | 17 | 18 |
| Cys | Ile | Met | Ala | Asn | Phe |
| 19 | 20 | | | | |
| Ser | Ala | | | | |

Determination:

A 14 µg portion of the FTX isolated by the above method of this invention was reduced and alkylated, and then its N- terminal amino acid sequence was determined by an automatic Edman degradation method using a Gas-Phase Protein Sequencer Model 470 A (mfd. by Applied Biosystems Co.). The result obtained confirms that a novel substance having the above sequence is provided by this invention which substance is different in amino acid sequence from previously known anti-tumor substances.

(f) Reactivity with antibodies against known substances:

The growth-inhibitory action of the FTX isolated by the above method of this invention was not neutralized by anti-TNF monoclonal antibody, anti-IFN-$\alpha$-$\beta$,$\gamma$ monoclonal antibody, and anti-IL-1 polyclonal antibody.

(g) Amino acid sequence estimated from DNA sequence.

The mRNA which coded the tumor cell inhibition factor of this invention is isolated from fetal human kidney-origin fibroblasts. Then, the gene is cloned, and the base sequence of the gene is determined.

The detailed method will be explained hereinafter.

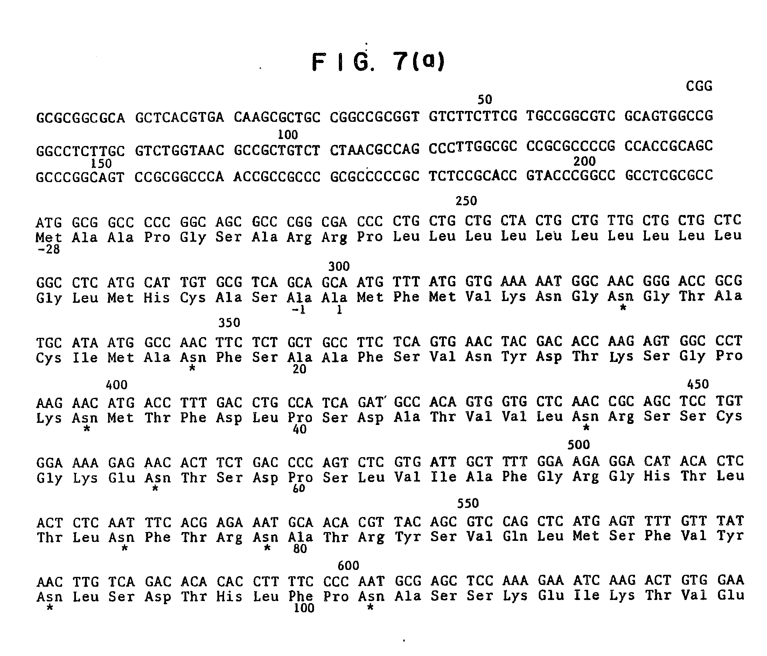

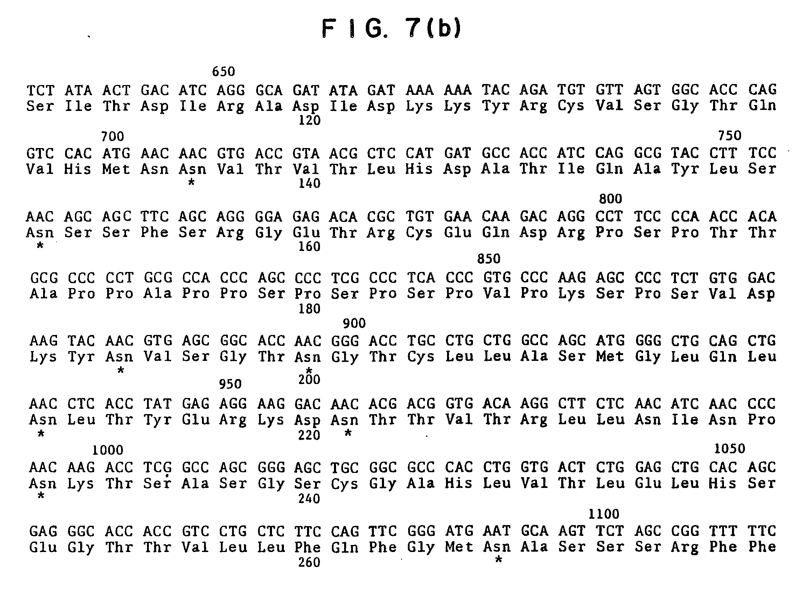

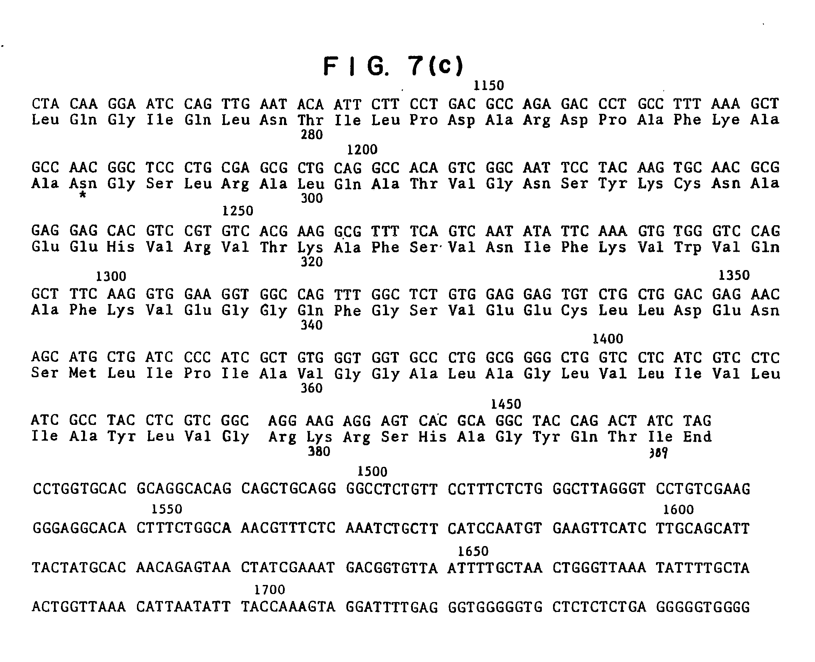

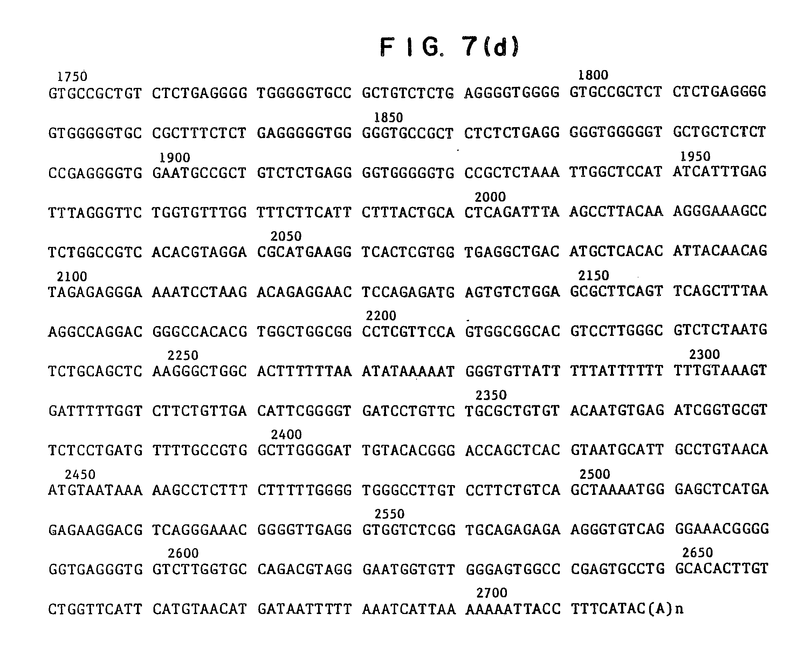

The base sequence and the amino acid sequence of intended polypeptide which is estimated from the base sequence are shown in Fig. 7. The base sequence consists of 1254 bases from the translation initiation signal ATG to the termination signal TAG. When translated into amino acids, it leads to a 417 amino acid sequence. The sequence of 20 amino acids from the 29th, Ala, to the 49th, Ala, entirely coincides with the amino acid sequence of the N-terminal of the polypeptide. It is considered that the amino acid acid sequence from the first, Met, to the 28th, Ala, is a signal sequence necessary for passage through cell membrane. The structural polypeptide estimated from the base sequence consists of 389 amino acids and contains 18 potential Asn linked glycosylation sites (Asn-X-Set/Thr).

Next, the substance of the present invention was fragmented by lysylendopeptides, and the fragments were purified by means of reverse high performance liquid chromatography. The purified fragments were analyzed with respect to amino acids contained, C-terminal peptide was identified in terms of existence or non-existence of a lysine residue, and the existence of a C-terminal amino acid of the C-terminal peptide is confirmed by effecting the digestion using carboxypeptidase Y. As the result, the natural-origin biological substance present was estimated as being constructed from two kinds of polypeptide, i.e., one having amino acids from N-terminal to 169th Ser and the other having the amino acids from N-terminal to 170th Pro, among the amino acid sequence of 389 amino acid residues estimated from the base sequence of the cDNA.

These findings strongly support the conclusion that the tumor cell inhibition factor of this invention comprises the following amino acid sequence.

| | | | | | | | | |
|---|---|---|---|---|---|---|---|---|
| Ala 1 | Met | Phe | Met | Val | Lys | Asn | Gly | Asn* | Gly |
| Thr | Ala | Cys | Ile | Met | Ala | Asn* | Phe | Ser | Ala 20 |
| Ala | Phe | Ser | Val | Asn | Tys | Asp | Thr | Lys | Ser |
| Gly | Pro | Lys | Asn* | Met | Thr | Phe | Asp | Leu | Pro 40 |
| Ser | Asp | Ala | Thr | Val | Val | Leu | Asn* | Arg | Ser |
| Ser | Cys | Gly | Lys | Glu | Asn* | Thr | Ser | Asp | Pro 60 |
| Ser | Leu | Val | Ile | Ala | Phe | Gly | Arg | Gly | His |
| Thr | Leu | Thr | Leu | Asn* | Phe | Thr | Arg | Asn* | Ala 80 |
| Thr | Arg | Tyr | Ser | Val | Gln | Leu | Met | Ser | Phe |
| Val | Tyr | Asn* | Leu | Ser | Asp | Thr | His | Leu | Phe 100 |
| Pro | Asn* | Ala | Ser | Ser | Lys | Glu | Ile | Lys | Thr |
| Val | Glu | Ser | Ile | Thr | Asp | Ile | Arg | Ala | Asp 120 |
| Ile | Asp | Lys | Lys | Tyr | Arg | Cys | Val | Ser | Gly |
| Thr | Gln | Val | His | Met | Asn | Asn* | Val | Thr | Val 140 |
| Thr | Leu | His | Asp | Ala | Thr | Ile | Gln | Ala | Tyr |
| Leu | Ser | Asn* | Ser | Ser | Phe | Ser | Arg | Gly | Glu 160 |
| Thr | Arg | Cys | Glu | Gln | Asp | Arg | Pro | Ser | -p |

(wherein p is OH or Pro, and the star marks indicate N-glucoside linkage sugar chain structure.)

8

(5) DNA sequence containing a DNA coding the present substance.

As starting cells, those of a human-origin established cell line, especially a human kidney-origin established cell line, are used. The DNA sequence containing a DNA sequence coding the substance of the present invention is obtained from either cDNA obtained from mRNA which has been extracted from the starting cells, or a library of chromosome DNA of the starting cells.

The amino acid sequence of the substance is estimated from the DNA sequence obtained. Further, this DNA sequence can be utilized in·the preparation of the substance of the invention according to gene recombination method.

① Extraction of poly(A)$^+$ RNA from fetal human kidney-origin fibroblast.

Cells are cultivated by using a serum-free medium obtained by adding 0.05 - 0.2% (w/v) of human serum albumin to the Waymouth medium, and then total RNA is prepared from $1.2 \times 10^{10}$ cultivated cells. Preferably it is performed by guanidine thiocyanate-cesium chloride method [Biochemistry, 18, 5294 (1979)-]. To cells are added 400 ml of a solution containing 6 M guanidine thiocyanate, 5 mM sodium citrate, 0.5% Sarkosyl, 0.1 M 2-mercaptoethanol and 0.1% Antifoam-A, and the whole is homogenized. Four grams of cesium chloride is dissolved for 10 ml of the homogenized solution. Ten milliliters of 5.7 M cesium chloride solution is placed in a polyallomer centrifuge tube, then 24 ml of the homogenized solution is placed thereon, and the resulting system is ultracentrifuged in a Beckman Ultracentrifuge (60 Ti rotor) at 35,000 rpm at 20°C for 16 hours. After centrifugation, the pellet is washed twice with 70% ethanol, and then heated in 10 ml of 10 mM tris-HCl buffer solution (pH 7.4) containing 5 mM EDTA and 1% SDS at 65°C for 5 minutes to form a solution. The solution is mixed with an equal amount of chloroform-n-butanol mixture (4 : 1, v/v). The resulting mixture is stirred and then centrifuged to obtain an aqueous phase. The aqueous phase is mixed with one tenth the amount of 3 M sodium acetate solution (pH 5.2) and 2.5 times the amount of ethanol. The mixture is allowed to stand at -70°C for 30 minutes and then centrifuged to obtain pellet. The pellet is dissolved in distilled water to obtain a total RNA solution. Poly(A)$^+$ RNA is purified from the total RNA by affinity chromatography utilizing the affinity for oligo ($\alpha$T). Oligo ($\alpha$T) cellulose is equilibrated with 10 mM tris-HCl buffer solution (pH 7.5), containing 1 mM EDTA, 0.5 M NaCl and 0.1% SDS. The total RNA is charged in the equilibrated column, and the adsorbed fraction is eluted with 10 mM tris-HCl buffer solution (pH 7.5) containing 1 mM EDTA and 0.05% SDS to obtain poly(A)$^+$ RNA. The poly-(A))$^+$ RNA is fractionated according to the dimension of RNA by means of sucrose density gradient centrifugation. The poly(A)$^+$ RNA solution is placed on 5% - 25% sucrose density gradient buffer solution (10 mM tris-HCl buffer solution (pH 7.5), containing 1 mM EDTA, 0.1 M NaCl, 0.1% SDS) and subjected to ultracentrifuge in a Beckman Ultracentrifuge (50.1 Ti rotor) at 20,000 rpm at 20°C for 16 hours, thus to be separated into 24 fractions.

② Synthesis of oligodeoxynucleotide

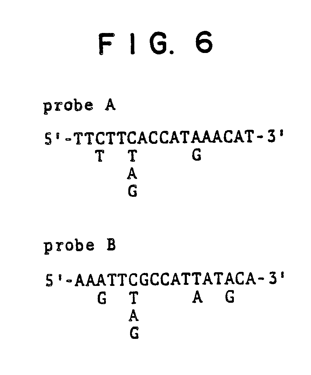

A hybridization method is used for cloning the intended gene. A preferably used probe for hybridization is a synthetic oligodeoxynucleotide. Two kinds of oligodioxynucleotide (17 bases) are synthesized which show complementarity to the base sequence estimated from the N-terminal amino acid sequence (see Fig. 6). A probe mixture comprising 16 kinds of complemental strand (probe A) to the base sequence corresponding to the sequence from Met, the second amino acid from the N-terminal, to Asn, the seventh from the N-terminal, on one hand, and a probe mixture comprising 48 kinds of complemental strand (probe B) to the base sequence corresponding to the sequence from the 13th, Cys, to the 18th, Phe, on the other hand are synthesized in a DNA synthesizer (Model 381 A, mfd. by Applied Biosystem Co.). The synthetic probes are used after labelling the 5'-terminal with T4 polynucleotide kinase (available from New England Biolabs) by using [$\gamma$-32P] ATP (available from Amersham).

③ Examination of the nucleic acid of the intended protein in poly(A)$^+$ RNA

By the northern blotting hybridization method, the size fractions of the poly(A)$^+$ RNA purified in ① above were examined to determine that in which the nucleic acid encoding the intended protein was present. The poly(A)$^+$ RNA is denatured. Preferably a formaldehyde method is used. The RNA is dissolved in a 10 mM sodium phosphate buffer (pH 6.8) containing 50% by volume of formamide, 6% by volume of formaldehyde, 0.2 mM EDTA, 10% of glycerine, and 0.0% (w/v) of BPB, and is denatured at 65°C for 5 minutes. The denatured RNA is subjected to electrophoresis using formaldehyde-agarose gel. The electrophored RNA is transferred from the gel to a Zeta Probe blotting membrane (available from Bio Rad) by means of a capillary method. The RNA adsorbed onto the membrane and the probe labelled in ② above are subjected to hybridization. The membrane is treated in a hybridization solution containing 6 x SSC (0.9 M NaCl, 90 mM sodium citrate) 20 mM sodium phosphate (pH 6.8), 7% SDS, 10 x Denhardt's (0.2% (w/v) Ficoll 400, 0.2% (w/v) polyvinylpyrrolidone, 0.2% (w/v) bovine serum albumin), 10% (w/v) dextran sulfate, 100 μg/ml of tRNA of yeast origin, and $^{32}$P-terminal labelled probed at 37°C overnight to effect hybridization, and then washed two times with washing solution containing 3 x SSC, 10 x Denhardt's, 5% SDS, 25 mM sodium phosphate buffer solution (pH 7.5) at 35°C for 30 minutes. The washed membrane is made to expose X-ray film for several days. The exposed X-ray film is developed and the size of the poly (A)$^+$ RNA hybridized with the labelled probe was determined. As a result, it was found that a band of a size of 2.7 kilobase (kb) appeared using both of the synthetic probes A and B.

④ Synthesis of cDNA

cDNA is synthesized from the fraction containing poly(A)$^-$ RNA having a size of 2.7 kilobase which had been hybridized with both of probes A and B. The synthesis of cDNA was performed by using a cDNA Synthesis System (Amersham) and according to the protocol. From 5 μg of poly(A)$^+$ RNA was obtained 3.3 μg of double stranded cDNA. Then, The cDNA library is prepared from the double stranded cDNA. Preferably, λ$_{gt}$ 10 is used as the vector of DNA. λ$_{gt}$ 10 forms a plaque of relatively large size and hence gives a good yield of recombinants. Before an EcoRI linker is connected to the cDNA, it is methylated by the use of EcoRI methylase with the aim of protecting the EcoRI site present inside the cDNA. A 1.5 μg portion of the double stranded cDNA is dissolved in a solution containing of 0.1 M NaCl, 0.1 M tris-HCl (pH 8.0), 1 mM EDTA, 80 μM S-adenosylmethionine, 30 units of EcoRI methylase (available from New England Biolabs) is added thereto, and the whole is allowed to react at 37°C for 60 minutes. The reaction mixture is extracted with phenol/chloroform and the cDNA is recovered from the extract by precipitation with ethanol. The recovered cDNA is allowed to react at 15°C overnight in a solution containing 66 mM tris-HCl (pH 7.5), 6.6 mM MgCl$_2$, 10 mM dithiothreitol, 0.1 mM ATP, and 1.5 μg of EcoRI linker (dGGAATTCC, available from New England Biolabs) by addition of 20 units of T4DNA ligase (available from New England Biolabs). The cDNA having the EcoRI linker connected thereto is digested with 150 units of EcoRI (available from Takara Shuzo Co.). The cDNA digested with EcoRI is separated from excess of the linker by means of Biogel A 50 m column chromatography. From 1.5 μg of the double stranded cDNA, was obtained 465 ng of double stranded cDNA having the EcoRI linker connected thereto. A 100 ng portion of the double stranded cDNA and 1 μg of λ$_{gt}$ 10 EcoRI arm are allowed to react at 15°C overnight in 5 μl of a reaction solution containing 66 mM tris-HCl (pH 7.5), 6.6 mM MgCl$_2$, 5 mM dithiothreitol, 1 m MATP, and 2.8 units of T4DNA ligase (available from Takara Shuzo Co.) to prepare recombinant DNA of λ$_{gt}$ 10 and cDNA. Then, a recombinant phage is prepared from the recombinant DNA by an in vitro packaging method. Preferably, an in vitro packaging kit of Gigapack gold (available from Strategene Co.) is used. The titer of the cDNA library was examined by using Escherichia coli c600 as an indicator strain. It was found that 3.9 x 10$^6$ plaques had been obtained.

⑤ Screening of cDNA

The intended cDNA is screened from the cDNA library obtained in ④ above by means of plaque hybridization using a $^{32}$P terminal-labelled probe. Preferably, the method of phage amplification on a filter (method in Enzymology, 68, 389, (1979)) is used. Escherichia coli c600 is infested with 2.4 x 10$^5$ recombinant phages, and allowed to form plaques on TB agar, which is then stored at 4°C. A colony plaque screen filter (NEN) is immersed in a suspension of Escherichia coli c600 to adsorb the bacteria on the filter, and then dried at room temperature. The filter is placed on Tb agar on which plaques have been formed, and is brought into contact therewith for several minutes. The filter is peeled off and placed on a new TB agar, which is then incubated overnight at 37°C. After incubation, the filter is peeled off, immersed in a solution containing of 0.5 M NaOH, 1.5 M NaCl for 5 minutes, further twice in 0.5 M tris-HCl buffer solution (pH 8.0) containing of 1.5 M NaCl for 5 minutes, and then in 2 x SSPE (0.36 NaCl, 20 mM $Na_2HPO_4$, 2 mM EDTA). Thereafter, it is dried at room temperature and then baked under vacuum at 80°C for 1 hour. The $^{32}$p terminal-labelled oligodeoxynucleotide fragments of B and A used in ③ above are employed as hybridization probes. The filter is immersed in a solution containing 6 x SSC (0.9 M NaCl, 90 mM sodium citrate), 20 mM sodium phosphate (pH 6.8), 10 x Denhardt's, 7% SDS, 10% dextran sulfate, 100 μg/ml of tRNA of Yeast origin, and 9 pmol $^{32}$p-terminal-labelled probe (specific activity: 5 x $10^6$ cpm/pmol), and incubated overnight at 37°C to effect hybridization. Thereafter, the filter is washed twice with 6 x SSC, then three times at 35°C for 30 minutes with washing solution containing a 3 x SSC, 10 x Denhardt's, 5% SDS, 25 mM sodium phosphate (pH 7.4) and then dried. Then, it is subjected to autoradiography. The plaques on the agar plate which correspond to the signal that has appeared at a position common to probes A and B are scratched off and suspended in a solution containing 0.1 M NaCl, 8.1 mM $MgSO_4$, 0.01% gelatin, 50 mM tris-HCl (pH 7.5). The plaque-forming reaction is again effected for the suspension to obtain 15 kinds of positive, single recombinant phages. Among these, phages whose cDNA have a size of 2.7 Kbp are selected to extract DNA therefrom, and the cDNA is introduced into the proliferation vector of Escherichia coli, preferably into pUC 18.

(6) Expression vector for the substance of this invention

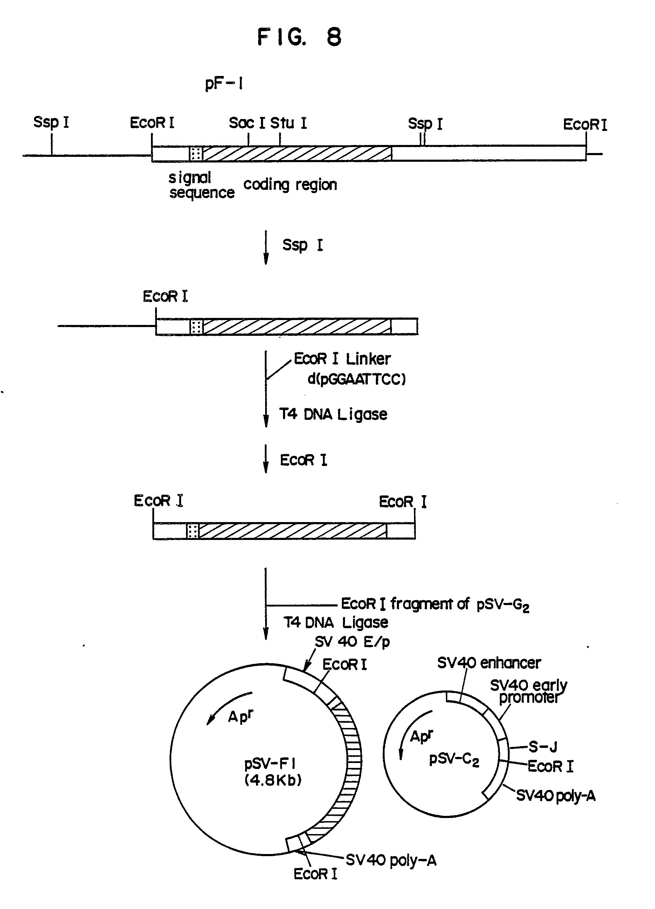

From a plasmid pF-1 in which the cDNA of the substance of this invention had been inserted at the EcoRI site of an Escherichia coli-cloning vector pUC 18, there was prepared a plasmid pSV-F1 in which a 1.7 kb fragment containing part of the 3′ non coding region, 5′-side non coding region, signal sequence and the coding region of the cDNA had been inserted at the EcoRI site of an animal cell expression vector $pSVG_2$ in positive direction (see Fig. 8).

The vector $pSVG_2$ possesses the enhancer, promoter, splicing junction, and poly A signal of SV 40 virus and contains necessary elements for expressing a useful substance in animal cells [cf. Japanese Patent Application Kokai (Laid-Open) No. 236,493/87]. The $pSVG_2$ was digested with EcoRI and further dephosphorylated at the 5′-terminal with alkaline phosphates of bovine small intestine origin. Then, the resulting product was subjected to 1% low-m.p. gel electrophoresis to recover DNA. The pF-1 which contained the whole cDNA of the substance of this invention was digested with SSpI and then subjected to 1% low-m.p. gel electrophoresis to recover 2.3 kb fragment. To the recovered DNA was connected an EcoRI linker (dpGGAATTCC, available from New England Biolabs). The recovered 2.3 kb fragment and the EcoRI linker were reacted with the aid of T4 DNA ligase (available from Takara Shuzo Co., Ltd.). The reaction product was further subjected to EcoRI digestion and then to 1% low-m.p. gel electrophoresis to recover 1.7 kb fragment. The 1.7 kb fragment and the EcoRI-digested $pSVG_2$ were reacted with the aid of T4 DNA ligase (available from Takara Shuzo Co., Ltd.) and transformed into Escherichia coli JM 109, thus to obtain an ampicillin-resistant transformant. From among the transformants, there was obtained pSV-F1 in which the 1.7 kb fragment had been inserted in positive direction.

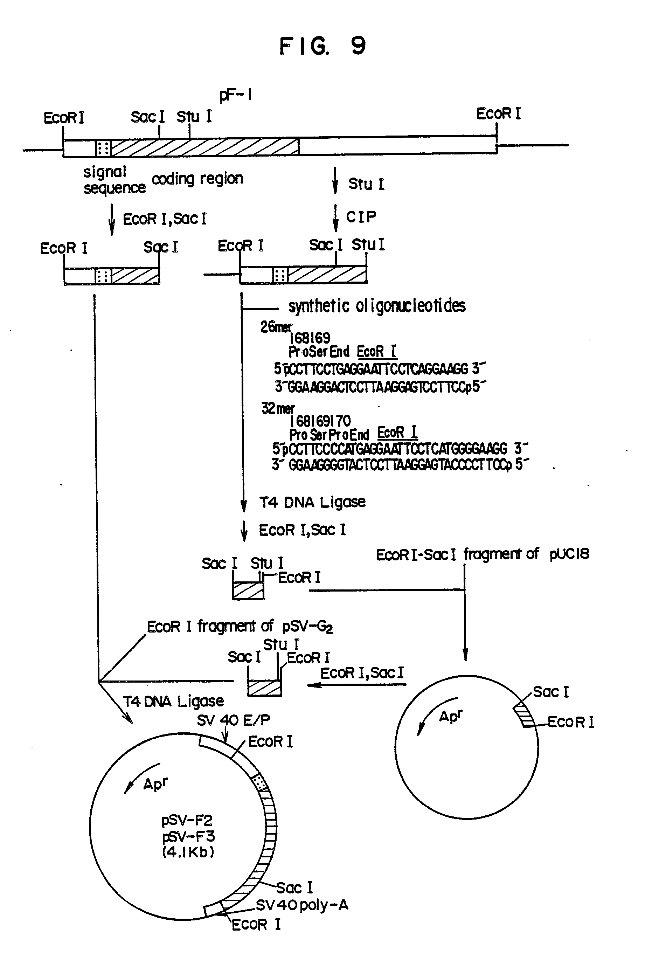

Since the substance of this invention of natural origin comprises peptide of from Ala of N-terminal to $^{169}$Ser or $^{170}$Pro of C-terminal, there were prepared expression plasmids pSV-F2 and pSV-F3 respectively capable of coding up to $^{169}$Ser and $^{170}$Pro (see Fig. 9). The pF-1 was digested with StuI which cuts the former at AGGCCT ($^{167}$Arg $^{168}$Pro), further subjected to dephosphorylation at the 5′-terminal with the aid of alkaline phosphates of bovine small intestine origin, and then subjected to 1% low m.p. gel electrophoresis, to recover DNA. To the StuI site of the recovered DNA, was connected a synthetic oligonucleotide. A synthetic oligonucleotide of 26 bases having termination codon TGA and EcoRI site at CCTTCC ($^{168}$Pro $^{169}$Ser) (5′CCTTCCTGAGGAATTCCTCAGGAAGG3′) and a synthetic oligonucleotide of 32 bases having termination codon TGA and EcoRI site at CCTTCCCCA ($^{168}$Pro $^{169}$Ser $^{170}$Pro) (5′CCTTCCCCATGAGGAATTCCTCATGGGGAAGG3′) were synthesized by the use of an automatic DNA synthesizer (mfd. by Applied Biosynthesis Co.). The 5′-terminal of the synthetic oligonucleotide of 26 mers and of 32 mers were phosphorylated with the aid of polynucleotide kinase (available from Takara Shuzo Co., Ltd.) and were connected with the StuI-digested pF-1 fragment with the aid of T4 DNA ligase (available from Takara Shuzo Co., Ltd.). The resulting product was then digested with EcoRI and SacI and subjected to 4% polyacrylamide gel electrophoresis to recover 0.2 kb fragment. The recovered SacI-EcoRI fragment was connected to the SacI-EcoRI site of cloning vector pUC 18 with the help of T4 DNA ligase (available from Takara Shuzo Co., Ltd.) and transformed into Escherichia coli JM 109. The base sequence of the plasmid insertion fragment of the transformant was determined by the dideoxy method and was confirmed to be the intended one. The above-mentioned plasmid was digested with SacI and EcoRI and then

11

subjected to 4% polyacrylamide gel electrophoresis to recover 0.2 kb fragment.

On the other hand, pF-I was digested with EcoRI and SacI to recover 0.61 kb fragment which contains 5′-side of 5′-side coding region, 5′-non coding region and signal sequence of the cDNA. The recovered EcoRI, SacI 0.61 kb fragment and SacI, EcoRI 0.2 kb fragment to which synthetic oligonucleotide had been connected were inserted to expression vector $pSVG_2$ digested with EcoRI, with the aid of $T_4$ DNA ligase and the resulting product was transformed into Escherichia coli JM 109. From the transformant there were obtained expression plasmids pSV-F2 and pSV-F3 which had been connected in intended direction.

(7) Transformed host cells

Host cells to be used preferably are Chinese hamster overy cells ($CHO-K_1$). Preferably used culture fluid is Ham's F-12 medium containing 10% of fetal bovine serum. Transfection of recombinant DNA into cells is performed by the calcium phosphate precipitation method (Strain et al. Biochem. J., 218, 475-482, 1984). To select a cell in which the present substance has been expressed, a method is employed which introduces a drug-resistance gene simultaneously to the cell and selecting the resulting drug-resistant strain (Sbraman, Anal. Biochem., 135, 1-15, 1983). In the present invention, there was used pSV-NEO formed by connecting to $pVSG_2$ and $Neo^r$ gene (aminoglycoside 3′-phospho-transferase II) which shows resistance to G-418 in animal cells (cf. Japanese Patent Application Kokai (Laid-Open) No. 236,493/87) (see Fig. 10).

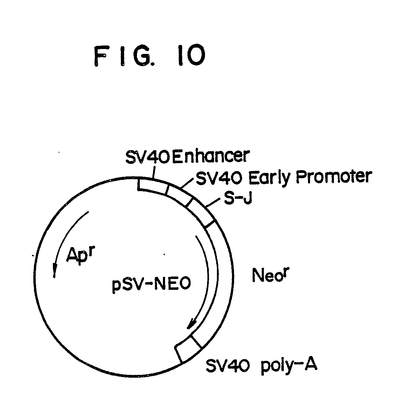

A 2.5 ml volume of HEPES buffer solution of double concentration (2 x HBS) [10 g/$\ell$ HEPES (N-(2-hydroxyethyl)piperazine-N′-2-ethanesulfonic acid), 6 g/$\ell$ NaCl, adjusted to pH 7.1], 50 $\mu$l of an aqueous solution of 70 mM $NaH_2PO_4$ and 70 mM $Na_2HPO_4$, 100 $\mu$l of 1 $\mu$g/ml salmon sperm DNA solution, and 100 $\mu$l of recombinant DNA [10 $\mu$g of pSV-F1 (pSV-F2, pSV-F3) and 0.1 $\mu$g of pSV-NEO (cf. Fig. 10)] were placed in a tube and made up with sterilized water to a total volume of 4.7 ml, thus to obtain B solution. To the B solution was added 300 $\mu$l of A solution (2 M $CaCl_2$). The A solution was slowly added dropwise to the B solution with stirring while air was being introduced into the B solution. After completion of the addition of A solution, introduction of air was continued for further 1 to 2 minutes to form a uniform precipitate. The resulting suspension was then allowed to stand for 10 minutes. $5 \times 10^5$ CHO cells were inoculated on a 100 mm Petri dish on the day preceeding transfection and cultivated in a $CO_2$ incubator for 24 hours. The culture fluid used was Ham's F-12 medium containing 10% fetal bovine serum. Onto the culture dish was added dropwise 1 ml of the microfine precipitate suspension and spread uniformly. The microfine precipitates of DNA-calcium phosphate were made to be adsorbed to the cells by allowing the mixture to stand still for 10 minutes. Then, the whole was again cultivated in a $CO_2$ incubator. Medium exchange was made 18 hours after transfection. After further 48 hours, the medium was replaced with a G-418-containing medium. The concentration of G-418 was 400 $\mu$g/ml. Colonies which had grown in the G-418- containing medium were separated by the cylinder method to obtain g-418 resistant transformant cells.

As the host cell, there may alternatively be used a human-origin cultivation-established fibroblast.

Brief Description of the Drawings

Fig. 1 shows graphs illustrating the gel filtration pattern of FTX obtained by using TSK Gel 3000 - 2000 SW. The upper graph shows a FTX activity pattern. The lower graph shows the light absorption of protein.

Fig. 2 is a graph showing the reversed phase HPLC pattern obtained by using RP-304 column. The symbol "FTX" indicated the substance of this invention.

Fig. 3 is a graph showing the inhibitory effect of FTX on the growth of KB cells. The KB cell growth inhibition rate (%) is calculated by the following equation.

$$\left(1 - \frac{A_{590} \text{ of FTX-treated cell}}{A_{590} \text{ of control cell}}\right) \times 100$$

Figs. 4 shows microphotographs (x 100) illustrating the growth inhibitory effect of FTX on KB cells. Fig. 4(b) is the microphotograph of the morphology of KB cells to which 4 U/ml of FTX has been added, and Fig. 4(a) is that of KB cells to which no FTX has been added.

Fig. 5 shows graphs illustrating the molecular weight determination of FTX by SDS polyacrylamide gel electrophoresis.

Fig. 5(a): Protein argentation band ... lane-1: marker proteins: lane-2: FTX (RP-304 step) 0.3 μg, reduced; lane-3: the same, but nonreduced.

Fig. 5(b): Activity of FTX extracted from a gel slice (5 mm/slice) ... FTX (TSK gel G3000 - 2000 SW step); 96 U/lane.

Fig. 6 is a chart showing the sequence of synthetic oligodeoxynucleotide used in scrutinizing the gene of the FTX of this invention.

Fig. 7 shows charts showing the base sequence of cDNA of FTX of this invention and the amino acid sequence of polypeptide estimated from the base sequence. The symbol * indicates an N-glucoside linkage sugar chain structure.

Fig. 8 is a diagram illustrating the preparation of pSV-F1.

Fig. 9 is a diagram illustrating the preparation of pSV-F2 and pSV-F3.

Fig. 10 is a diagram illustrating the selection plasmid pSV-NEO used in transfection, wherein $Ap^r$ is an ampicillin-resistant gene, CIP is a bovine small intestine-origin alkaline phosphatase, S-J is a splicing junction, $NeO^r$ is a neomycin-resistant gene, and SV 40 poly-A is Poly-A addition signal of SV 40.

Having elucidated the DNA sequence and expressed it in a recombinant form to produce a protein, it will be apparent to those skilled in the art that various modifications to the nature of the protein can be made while retaining the essential functional characteristics of tumor cell inhibition factor.

Such modifications may for example comprise amino acid deletions, insertions, additions, substitutions, replacements and inversions; and also alteration to or elimination of any glycosylation.

It will also be apparent to those skilled in the art using the information disclosed herein that DNA encoding the polypeptide products thereof can now be obtained by routine methods such as direct DNA synthesis, or isolation and cloning of cDNA using specific oligonucleotide probes for selected regions of the sequence disclosed herein, or combinations of these. If desired, suitable encoding DNA can be isolated from a genomic library using such oligonucleotide probes.

In addition to the possibility of specifically engineered modifications, natural allelic variants may arise, depending for example upon the source of the DNA.

**Claims**

1. A tumor cell inhibition factor which is a glycoprotein obtainable from a culture supernatant of human-origin fibroblasts and which has the following characteristic properties:

(a) molecular weight: single band of 36,000 ± 1,000 daltons as determined by SDS-polyacrylamide gel electrophoresis under reduced and nonreduced conditions,

(b) Isoelectric point: pI of at least 10.5,

(c) Affinity for concanavalin A: adsorption,

(d) N-terminal amino acid sequence:

Ala - Met - Phe - Met

- Val - Lys - Asn - Gly

- Asn - Gly - Thr - Ala

- Cys - Ile - Met - Ala

- Asn - Phe - Ser - Ala

(e) Amino acid composition:

13

| AMINO ACID | P mole | mole % |
|---|---|---|
| Asp | 773.56 | 9.25 |
| Glu | 395.42 | 4.72 |
| Ser | 947.61 | 11.32 |
| Gly | 447.52 | 5.35 |
| His | 284.40 | 3.39 |
| Arg | 354.72 | 4.24 |
| Thr | 806.66 | 9.64 |
| Ala | 981.16 | 11.72 |
| Pro | 308.71 | 3.69 |
| Tyr | 305.52 | 3.66 |
| Val | 603.42 | 7.20 |
| Met | 337.81 | 4.04 |
| Cys | ND | - |
| Ile | 360.89 | 4.31 |
| Leu | 558.08 | 6.67 |
| Phe | 494.43 | 5.90 |
| Trp | ND | - |
| Lis | 412.36 | 4.92 |
| TOTAL | 8372.27 | 100.00 |

Amount of the substance applied: 5 μg

ND: not determined

(f) Biological activity: It exerts growth inhibition and degeneration effects at least on KB cells of rhinopharyngeal cancer origin (ATCC CCL-17),

(g) Cross reactivity: It is not neutralized with an anti-TNF monoclonal anitbody, anti-interferon α-, β- and γ-monoclonal antibody, and an anti-IL-1 polyclonal antibody.

2. A DNA sequence which encodes a polypeptide comprising the following amino acid sequence

| Ala | Met | Phe | Met | Val | Lys | Asn | Gly | Asn | Gly |
|-----|-----|-----|-----|-----|-----|-----|-----|-----|-----|
| 1 | | | | | | | | | |
| Thr | Ala | Cys | Ile | Met | Ala | Asn | Phe | Ser | Ala |
| | | | | | | | | | 20 |
| Ala | Phe | Ser | Val | Asn | Tys | Asp | Thr | Lys | Ser |
| Gly | Pro | Lys | Asn | Met | Thr | Phe | Asp | Leu | Pro |
| | | | | | | | | | 40 |
| Ser | Asp | Ala | Thr | Val | Val | Leu | Asn | Arg | Ser |
| Ser | Cys | Gly | Lys | Glu | Asn | Thr | Ser | Asp | Pro |
| | | | | | | | | | 60 |
| Ser | Leu | Val | Ile | Ala | Phe | Gly | Arg | Gly | His |
| Thr | Leu | Thr | Leu | Asn | Phe | Thr | Arg | Asn | Ala |
| | | | | | | | | | 80 |
| Thr | Arg | Tyr | Ser | Val | Gln | Leu | Met | Ser | Phe |
| Val | Tyr | Asn | Leu | Ser | Asp | Thr | His | Leu | Phe |
| | | | | | | | | | 100 |
| Pro | Asn | Ala | Ser | Ser | Lys | Glu | Ile | Lys | Thr |
| Val | Glu | Ser | Ile | Thr | Asp | Ile | Arg | Ala | Asp |
| | | | | | | | | | 120 |
| Ile | Asp | Lys | Lys | Tyr | Arg | Cys | Val | Ser | Gly |
| Thr | Gln | Val | His | Met | Asn | Asn | Val | Thr | Val |
| | | | | | | | | | 140 |
| Thr | Leu | His | Asp | Ala | Thr | Ile | Gln | Ala | Tyr |
| Leu | Ser | Asn | Ser | Ser | Phe | Ser | Arg | Gly | Glu |
| | | | | | | | | | 160 |
| Thr | Arg | Cys | Glu | Gln | Asp | Arg | Pro | Ser | -P |

(wherein P denotes OH or Pro.)

15

or an allele or modification of the polypeptide having characteristics of tumor cell inhibition factor.

3. A DNA sequence according to Claim 2 wherein the DNA sequence which codes the tumor cell inhibition factor is represented by the base sequence of from base number 298 up to and including base number 804 or 807 shown in Fig. 7.

4. An expression vector capable of expressing in a suitable host cell the DNA sequence of claim 2 or claim 3.

5. Recombinant host cells or host cell cultures transformed with the vector of Claim 4.

6. A process for producing the tumor cell inhibition factor of Claim 1, which comprisies cultivating an established cell line obtained from a fetal human kidney or other suitable cell source, removing contaminants from the supernatant of the culture fluid and obtaining a fraction containing a substance having a molecular weight of 10,000 or more, dialysing the fraction obtained and passing the treated fraction through an anion exchanger, subjecting the unabsorbed fraction to a chromatofocussing treatment on an anion exchanger, subjecting the unabsorbed fraction to ultrafiltration on a membrane of molecular weight cut-off level of 10,000 to collect a high molecular weight fraction, and subjecting the high molecular weight fraction to gel filtration by high performance liquid chromatography to obtain an active protein fraction peak between MW 67 K and 32 K to recover an active ingredient.

7. A process according to Claim 6, wherein the active protein fraction is subjected to a reverse phase high performance liquid chromatography to obtain an active ingredient in pure form.

8. A polypeptide comprising the amino acid sequence as defined in Claim 2, or an allele or modification thereof having characteristics of tumor cell inhibition factor.

9. A process for producing a polypeptide of Claim 8, which comprises expressing in a recombinant host cell a DNA insert encoding said polypeptide.

10. A polypeptide to Claim 8 for use as a medicine.

Claims for the following contracting State: ES

1. A process for obtaining tumor cell inhibition factor which comprises isolating from a culture supernatant of human-origin fibroblasts, a glycoprotein which has the following characteristic properties:

(a) molecular weight: single band of 36,000 ± 2,000 daltons as determined by SDS-polyacrylamide gel electrophoresis under reduced and nonreduced conditions,

(b) Isoelectric point: pl of at least 10.5,

(c) Affinity for concanavalin A: adsorption,

(d) N-terminal amino acid sequence:

$$Ala - Met - Phe - Met$$

$$- Val - Lys - Asn - Gly$$

$$- Asn - Gly - Thr - Ala$$

$$- Cys - Ile - Met - Ala$$

$$- Asn - Phe - Ser - Ala$$

(e) Amino acid composition:

| AMINO ACID | P mole | mole % |
|---|---|---|
| Asp | 773.56 | 9.25 |
| Glu | 395.42 | 4.72 |
| Ser | 947.61 | 11.32 |
| Gly | 447.52 | 5.35 |
| His | 284.40 | 3.39 |
| Arg | 354.72 | 4.24 |
| Thr | 806.66 | 9.64 |
| Ala | 981.16 | 11.72 |
| Pro | 308.71 | 3.69 |
| Tyr | 305.52 | 3.66 |
| Val | 603.42 | 7.20 |
| Met | 337.81 | 4.04 |
| Cys | ND | - |
| Ile | 360.89 | 4.31 |
| Leu | 558.08 | 6.67 |
| Phe | 494.43 | 5.90 |
| Trp | ND | - |
| Lis | 412.36 | 4.92 |
| TOTAL | 8372.27 | 100.00 |

Amount of the substance applied: 5 $\mu$g
ND: not determined

(f) Biological activity: It exerts growth inhibition and degeneration effects at least on KB cells of rhinopharyngeal cancer origin (ATCC CCL-17),

(g) Cross reactivity: It is not neutralized with an anti-TNF monoclonal antibody, anti-interferon $\alpha$-, $\beta$- and $\gamma$-monoclonal antibody, and an anti-IL-1 polyclonal antibody.

2. A process according to Claim 1, which comprises cultivating an established cell line obtained from a fetal human kidney or other suitable cell source,

removing contaminants from the supernatant of the culture fluid and obtaining a fraction containing a substance having a molecular weight of 10,000 or more, dialysing the fraction obtained and passing the treated fraction through an anion exchanger,

subjecting the unabsorbed fraction to a chromatofocussing treatment on an anion exchanger, subjecting the unabsorbed fraction to ultrafiltration on a membrane of molecular weight cut-off level of 10,000, to collect a high molecular weight fraction, and

subjecting the high molecular weight fraction to gel filtration by high performance liquid chromatography to obtain an active protein fraction peak between MW 67 K and 32 K to recover an active ingredient.

3. A process according to Claim 7, wherein the active protein fraction is subjected to a reverse phase high performance liquid chromatography to obtain an active ingredient in pure form.

17

F I G. 1

F I G.  2

# F I G. 3

FIG. 4(a)

X 100

FIG. 4(b)

X 100

## F I G. 5(a)

## F I G. 5(b)

EP 0 322 084 A2

# F I G. 6

probe A

```
5'-TTCTTCACCATAAACAT-3'
     T   T       G
         A
         G
```

probe B

```
5'-AAATTCGCCATTATACA-3'
     G   T     A   G
         A
         G
```

# FIG. 7(a)

```
                                                                    CGG
                                       50
GCGCGGCGCA GCTCACGTGA CAAGCGCTGC CGGCCGCGGT GTCTTCTTCG TGCCGGCGTC GCAGTGGCCG
                              100
GGCCTCTTGC GTCTGGTAAC GCCGCTGTCT CTAACGCCAG CCCTTGGCGC CCGCGCCCCG CCACCGCAGC
    150                                                           200
GCCCGGCAGT CCGCGGCCCA ACCGCCGCCC GCGCCCCCGC TCTCCGCACC GTACCCGGCC GCCTCGCGCC

                                               250
ATG GCG GCC CCC GGC AGC GCC CGG CGA CCC CTG CTG CTG CTA CTG CTG TTG CTG CTG CTC
Met Ala Ala Pro Gly Ser Ala Arg Arg Pro Leu Leu Leu Leu Leu Leu Leu Leu Leu Leu
-28
                                   300
GGC CTC ATG CAT TGT GCG TCA GCA GCA ATG TTT ATG GTG AAA AAT GGC AAC GGG ACC GCG
Gly Leu Met His Cys Ala Ser Ala Ala Met Phe Met Val Lys Asn Gly Asn Gly Thr Ala
                        -1   1                                *
                350
TGC ATA ATG GCC AAC TTC TCT GCT GCC TTC TCA GTG AAC TAC GAC ACC AAG AGT GGC CCT
Cys Ile Met Ala Asn Phe Ser Ala Ala Phe Ser Val Asn Tyr Asp Thr Lys Ser Gly Pro
                    *        20
    400                                                               450
AAG AAC ATG ACC TTT GAC CTG CCA TCA GAT GCC ACA GTG GTG CTC AAC CGC AGC TCC TGT
Lys Asn Met Thr Phe Asp Leu Pro Ser Asp Ala Thr Val Val Leu Asn Arg Ser Ser Cys
        *                40                                    *
                                                          500
GGA AAA GAG AAC ACT TCT GAC CCC AGT CTC GTG ATT GCT TTT GGA AGA GGA CAT ACA CTC
Gly Lys Glu Asn Thr Ser Asp Pro Ser Leu Val Ile Ala Phe Gly Arg Gly His Thr Leu
            *                60
                                          550
ACT CTC AAT TTC ACG AGA AAT GCA ACA CGT TAC AGC GTC CAG CTC ATG AGT TTT GTT TAT
Thr Leu Asn Phe Thr Arg Asn Ala Thr Arg Tyr Ser Val Gln Leu Met Ser Phe Val Tyr
            *               *   80
                                      600
AAC TTG TCA GAC ACA CAC CTT TTC CCC AAT GCG AGC TCC AAA GAA ATC AAG ACT GTG GAA
Asn Leu Ser Asp Thr His Leu Phe Pro Asn Ala Ser Ser Lys Glu Ile Lys Thr Val Glu
    *                       100       *
```

EP 0 322 084 A2

# F I G. 7(b)

```
                         650
TCT ATA ACT GAC ATC AGG GCA GAT ATA GAT AAA AAA TAC AGA TGT GTT AGT GGC ACC CAG
Ser Ile Thr Asp Ile Arg Ala Asp Ile Asp Lys Lys Tyr Arg Cys Val Ser Gly Thr Gln
                             120
    700                                                                750
GTC CAC ATG AAC AAC GTG ACC GTA ACG CTC CAT GAT GCC ACC ATC CAG GCG TAC CTT TCC
Val His Met Asn Asn Val Thr Val Thr Leu His Asp Ala Thr Ile Gln Ala Tyr Leu Ser
                     *           140
                                                         800
AAC AGC AGC TTC AGC AGG GGA GAG ACA CGC TGT GAA CAA GAC AGG CCT TCC CCA ACC ACA
Asn Ser Ser Phe Ser Arg Gly Glu Thr Arg Cys Glu Gln Asp Arg Pro Ser Pro Thr Thr
 *                       160
                                             850
GCG CCC CCT GCG CCA CCC AGC CCC TCG CCC TCA CCC GTG CCC AAG AGC CCC TCT GTG GAC
Ala Pro Pro Ala Pro Pro Ser Pro Ser Pro Ser Pro Val Pro Lys Ser Pro Ser Val Asp
                         180
                             900
AAG TAC AAC GTG AGC GGC ACC AAC GGG ACC TGC CTG CTG GCC AGC ATG GGG CTG CAG CTG
Lys Tyr Asn Val Ser Gly Thr Asn Gly Thr Cys Leu Leu Ala Ser Met Gly Leu Gln Leu
                 *               *
                     950     200
AAC CTC ACC TAT GAG AGG AAG GAC AAC ACG ACG GTG ACA AGG CTT CTC AAC ATC AAC CCC
Asn Leu Thr Tyr Glu Arg Lys Asp Asn Thr Thr Val Thr Arg Leu Leu Asn Ile Asn Pro
 *                       220   *
    1000                                                            1050
AAC AAG ACC TCG GCC AGC GGG AGC TGC GGC GCC CAC CTG GTG ACT CTG GAG CTG CAC AGC
Asn Lys Thr Ser Ala Ser Gly Ser Cys Gly Ala His Leu Val Thr Leu Glu Leu His Ser
 *                       240
                                     1100
GAG GGC ACC ACC GTC CTG CTC TTC CAG TTC GGG ATG AAT GCA AGT TCT AGC CGG TTT TTC
Glu Gly Thr Thr Val Leu Leu Phe Gln Phe Gly Met Asn Ala Ser Ser Ser Arg Phe Phe
                         260             *
```

EP 0 322 084 A2

# F I G. 7(c)

```
                                                    1150
CTA CAA GGA ATC CAG TTG AAT ACA ATT CTT CCT GAC GCC AGA GAC CCT GCC TTT AAA GCT
Leu Gln Gly Ile Gln Leu Asn Thr Ile Leu Pro Asp Ala Arg Asp Pro Ala Phe Lye Ala
                            280
                                    1200
GCC AAC GGC TCC CTG CGA GCG CTG CAG GCC ACA GTC GGC AAT TCC TAC AAG TGC AAC GCG
Ala Asn Gly Ser Leu Arg Ala Leu Gln Ala Thr Val Gly Asn Ser Tyr Lys Cys Asn Ala
        *                       300
                    1250
GAG GAG CAC GTC CGT GTC ACG AAG GCG TTT TCA GTC AAT ATA TTC AAA GTG TGG GTC CAG
Glu Glu His Val Arg Val Thr Lys Ala Phe Ser Val Asn Ile Phe Lys Val Trp Val Gln
                            320
        1300                                                            1350
GCT TTC AAG GTG GAA GGT GGC CAG TTT GGC TCT GTG GAG GAG TGT CTG CTG GAC GAG AAC
Ala Phe Lys Val Glu Gly Gly Gln Phe Gly Ser Val Glu Glu Cys Leu Leu Asp Glu Asn
                            340
                                            1400
AGC ATG CTG ATC CCC ATC GCT GTG GGT GGT GCC CTG GCG GGG CTG GTC CTC ATC GTC CTC
Ser Met Leu Ile Pro Ile Ala Val Gly Gly Ala Leu Ala Gly Leu Val Leu Ile Val Leu
                            360
                                        1450
ATC GCC TAC CTC GTC GGC  AGG AAG AGG AGT CAC GCA GGC TAC CAG ACT ATC TAG
Ile Ala Tyr Leu Val Gly  Arg Lys Arg Ser His Ala Gly Tyr Gln Thr Ile End
                    380                                 389
                1500
CCTGGTGCAC GCAGGCACAG CAGCTGCAGG GGCCTCTGTT CCTTTCTCTG GGCTTAGGGT CCTGTCGAAG
        1550                                                1600
GGGAGGCACA CTTTCTGGCA AACGTTTCTC AAATCTGCTT CATCCAATGT GAAGTTCATC TTGCAGCATT
                                    1650
TACTATGCAC AACAGAGTAA CTATCGAAAT GACGGTGTTA ATTTTGCTAA CTGGGTTAAA TATTTTGCTA
                1700
ACTGGTTAAA CATTAATATT TACCAAAGTA GGATTTTGAG GGTGGGGGTG CTCTCTCTGA GGGGGTGGGG
```

EP 0 322 084 A2

# F I G. 7(d)

GTGCCGCTGT CTCTGAGGGG TGGGGGTGCC GCTGTCTCTG AGGGGTGGGG GTGCCGCTCT CTCTGAGGGG

GTGGGGGTGC CGCTTTCTCT GAGGGGGTGG GGGTGCCGCT CTCTCTGAGG GGGTGGGGGT GCTGCTCTCT

CCGAGGGGTG GAATGCCGCT GTCTCTGAGG GGTGGGGGTG CCGCTCTAAA TTGGCTCCAT ATCATTTGAG

TTTAGGGTTC TGGTGTTTGG TTTCTTCATT CTTTACTGCA CTCAGATTTA AGCCTTACAA AGGGAAAGCC

TCTGGCCGTC ACACGTAGGA CGCATGAAGG TCACTCGTGG TGAGGCTGAC ATGCTCACAC ATTACAACAG

TAGAGAGGGA AAATCCTAAG ACAGAGGAAC TCCAGAGATG AGTGTCTGGA GCGCTTCAGT TCAGCTTTAA

AGGCCAGGAC GGGCCACACG TGGCTGGCGG CCTCGTTCCA GTGGCGGCAC GTCCTTGGGC GTCTCTAATG

TCTGCAGCTC AAGGGCTGGC ACTTTTTTAA ATATAAAAAT GGGTGTTATT TTTATTTTTT TTTGTAAAGT

GATTTTTGGT CTTCTGTTGA CATTCGGGGT GATCCTGTTC TGCGCTGTGT ACAATGTGAG ATCGGTGCGT

TCTCCTGATG TTTTGCCGTG GCTTGGGGAT TGTACACGGG ACCAGCTCAC GTAATGCATT GCCTGTAACA

ATGTAATAAA AAGCCTCTTT CTTTTTGGGG TGGGCCTTGT CCTTCTGTCA GCTAAAATGG GAGCTCATGA

GAGAAGGACG TCAGGGAAAC GGGGTTGAGG GTGGTCTCGG TGCAGAGAGA AGGGTGTCAG GGAAACGGGG

GGTGAGGGTG GTCTTGGTGC CAGACGTAGG GAATGGTGTT GGGAGTGGCC CGAGTGCCTG GCACACTTGT

CTGGTTCATT CATGTAACAT GATAATTTTT AAATCATTAA AAAAATTACC TTTCATAC(A)n

# F I G. 8

# FIG. 9

# F I G. 10

SV40 Enhancer
SV40 Early Promoter
S-J
Ap$^r$
pSV-NEO
Neo$^r$
SV40 poly-A